# EUROPÄISCHE PATENTANMELDUNG

(11) **EP 1 116 717 A1**
(43) Veröffentlichungstag der Anmeldung: **18.07.2001**
(21) Anmeldenummer: 01107592.6
(22) Anmeldetag: 10.05.1995
(51) Int. Cl.: C07D 231/20, A01N 43/56, C07D 231/18

(54) **Substituierte 3-Phenylpyrazole als Herbizide**

(30) Priorität: 20.05.1994 DE 4417837
(62) Teilanmeldung aus: 95920017.1
(71) Anmelder: BASF AKTIENGESELLSCHAFT, 67056 Ludwigshafen (DE)
(72) Erfinder: Von dem Bussche-Hünnefeld, Christoph-Sweder, 68199 Mannheim (DE); Klintz, Ralf, 67269 Gruenstadt (DE); Hamprecht, Gerhard, 69469 Weinheim (DE); Heistracher, Elisabeth, 67061 Ludwigshafen (DE); Schäfer, Peter, 67308 Ottersheim (DE); Münster, Peter, 67354 Römerberg (DE); Ditrich, Klaus, 67161 Gönnheim (DE); König, Hartmann, 69115 Heidelberg (DE); Westphalen, Karl-Otto, 67346 Speyer (DE); Gerber, Matthias, 67117 Limburgerhof (DE); Walter, Helmut, 67283 Obrigheim (DE)

(57) **Zusammenfassung**

Substituierte 3-Phenylpyrazole der Formel I 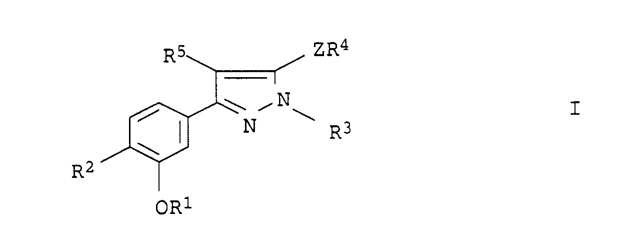 sowie deren landwirtschaftlich brauchbaren Salze, wobei
- R² =: CN, CF₃, Halogen;
- R³ =: H, C₁-C₄-Alkyl, C₁-C₄-Halogenalkyl;
- R⁴ =: C₁-C₄-Alkyl, C₁-C₄-Halogenalkyl;
- R⁵ =: H, NO₂, Halogen, -COOR²⁹, geg. subst. Aminocarbonyl;
- Z =: -O-, -S-, -SO-, -SO₂-;
- R¹,R²⁹ =: H, C₁-C₈-Alkyl, C₁-C₈-Halogenalkyl, C₃-C₆-Alkenyl, geg. subst. C₃-C₇-Cycloalkyl oder C₅-C₇-Cycloalkenyl, C₃-C₆-Halogenalkenyl, Cyano-C₁-C₈-alkyl, C₃-C₆-Alkinyl, C₁-C₄-Alkoxy-C₁-C₄-alkyl, 2-Tetrahydrofuranyl-C₁-C₈-alkyl, 3-Oxetanyl, 3-Thiethanyl, Carboxyl-C₁-C₆-alkyl, C₁-C₈-Alkoxycarbonyl-C₁-C₆-alkyl, C₁-C₄-Alkoxy-C₁-C₄-alkoxycarbonyl-C₁-C₆-alkyl, Cyclopropylmethyl, (1-Methylthiocyclopropyl)methyl, C₃-C₉-(α-Alkylalkyliden)iminooxy-C₁-C₆-alkyl, C₁-C₄-Alkylcarbonyl, Alkoxy-, Halogenalkoxy-, Alkenyloxy-, Halogenalkenyloxy- oder Arylalkoxyiminoalkyl, geg. subst. Phenyl, Phenyl-C₁-C₆-alkyl, Phenyl-C₂-C₆-alkenyl, Phenyl-C₃-C₆-alkinyl, Phenoxy-C₁-C₆-alkyl, Heteroaryl, Heteroaryl-C₁-C₆-alkyl, Heteroaryl-C₃-C₆-alkenyl, Heteroaryl-C₃-C₆-alkinyl oder Heteroaryloxy-C₁-C₆-alkyl;

Verwendung: als Herbizide.

## Beschreibung

Die vorliegende Erfindung betrifft neue substituierte 3-Phenylpyrazole der Formel I 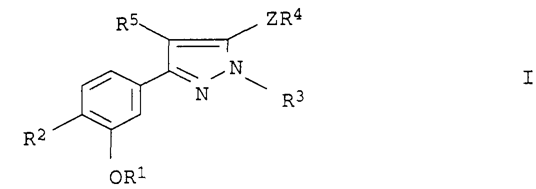 in der die Variablen folgende Bedeutung haben:
- R²: Cyano, Trifluormethyl oder Halogen;
- R³: Wasserstoff, C₁-C₄-Alkyl oder C₁-C₄-Halogenalkyl;
- R⁴: C₁-C₄-Alkyl oder C₁-C₄-Halogenalkyl;
- R⁵: Wasserstoff, Nitro, Halogen, -COOR²⁹ oder -CO-N(R³⁰,R³¹);
- z: Sauerstoff, Schwefel, -SO- oder -SO₂-;
- X,Y: unabhängig voneinander Sauerstoff oder Schwefel;
- A: eine chemische Bindung, Methylen, Ethylen, 1,3-Propylen, 1,4-Butylen, Vinylen oder 1,4-Butadienylen;
- R¹, R²⁹: unabhängig voneinander
Wasserstoff, C₁-C₈-Alkyl, C₁-C₈-Halogenalkyl, C₃-C₇-Cycloalkyl, das seinerseits ein bis drei C₁-C₃-Alkylreste tragen kann, C₃-C₆-Alkenyl, C₅-C₇-Cycloalkenyl, das seinerseits ein bis drei C₁-C₃-Alkylreste tragen kann, C₃-C₆-Halogenalkenyl, Cyano-C₁-C₈-alkyl, C₃-C₆-Alkinyl, C₁-C₄-Alkoxy-C₁-C₄-alkyl, 2-Tetrahydrofuranyl-C₁-C₈-alkyl, 3-Oxetanyl, 3-Thiethanyl, Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, Cyclopropylmethyl, (l-Methylthiocycloprop-l-yl)methyl, C₃-C₉-(α-Alkylalkyliden)iminooxy-C₁-C₆-alkyl, (C₁-C₄-Alkyl)carbonyl, C₁-C₄-Alkyl, das durch -C(R¹⁹)=N-O-(C₁-C₄-Alkyl), -C(R¹⁹)=N-O- (C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl)-R³⁴ substituiert ist, Phenyl, Phenyl-C₁-C₆-alkyl, Phenyl-C₂-C₆-alkenyl, Phenyl-C₃-C₆-alkinyl oder Phenoxy-C₁-C₆-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl, Heteroaryl-C₁-C₆-alkyl, Heteroaryl-C₃-C₆-alkenyl, Heteroaryl-C₃-C₆-alkinyl oder Heteroaryloxy-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ringglied einen Rest tragen kann, jeweils ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
- R³⁰, R³¹: unabhängig voneinander
Wasserstoff, C₁-C₈-Alkyl, C₁-C₈-Halogenalkyl, C₂-C₈-Alkenyl, C₂-C₈-Alkinyl, C₁-C₄-Alkoxy-C₁-C₄-alkyl, C₁-C₄-Alkylthio-C₁-C₄-alkyl, Cyano-C₁-C₈-alkyl, Carboxyl-C₁-C₄-alkyl, (C₁-C₄-Alkoxy) carbonyl-C₁-C₄-alkyl, C₁-C₄-Alkylsulfonyl-C₁-C₄-Alkyl, C₃-C₈-Cycloalkyl, C₁-C₆-Alkoxy, (C₃-C₆-Cycloalkoxy) carbonyl-C₁-C₄-alkyl, C₁-C₄-Alkoxy-(C₁-C₄-alkoxy)carbonyl-C₁-C₄-alkyl, (C₁-C₄-Alkyl)carbonyl, (C₁-C₄-Halogenalkyl) carbonyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
oder
- R³⁰ und R³¹: zusammen eine Tetramethylen-, Pentamethylen- oder Ethylenoxyethylenkette, die gewünschtenfalls ein bis drei C₁-C₄-Alkylreste und/ oder einen Rest -COOR⁶ tragen kann;
- R¹⁹: Wasserstoff, C₁-C₄-Alkyl, Phenyl oder Benzyl;
- R³⁴: Phenyl oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei jeder Phenyl- oder Heteroarylring unsubstituiert sein oder an jedem substituierbaren Ringglied gewünschtenfalls einen Substituenten tragen kann, jeweils ausgewählt aus der Gruppe bestehend aus Hydroxy, Nitro, Cyano, Halogen, C₁-C₄-Alkyl, C₁-C₄-Halogenalkyl, C₁-C₄-Alkoxy und C₁-C₄-Alkylthio,
sowie die landwirtschaftlich brauchbaren Salze von I.

Außerdem betrifft die Erfindung die Verwendung dieser Verbindungen als Herbizide, herbizide Mittel, welche die Verbindungen I als wirksame Substanzen enthalten, Verfahren zur Herstellung dieser herbiziden Mittel sowie Verfahren zur Bekämpfung von unerwünschtem Pflanzenwuchs mit den Verbindungen I.

In der JP O3/151 367 werden herbizid wirksame 1-(1-Alkyl-4-halogen-5-halogenalkoxy-lH-pyrazol-3-yl)-4,6-dihalogenphenyl-Derivate mit verschiedenen Substituenten in 3-Position des Phenylrings beschrieben, u.a. Verbindungen mit dem folgenden Substitutionsmuster IIa: 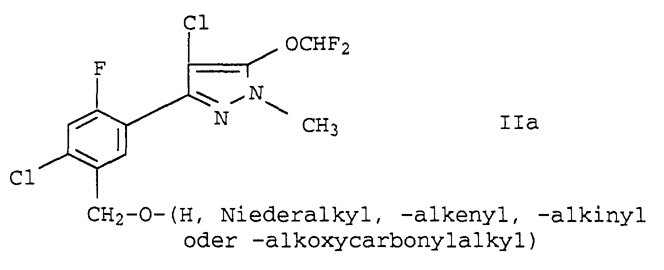 Außerdem wird in der EP-A 443 059 gelehrt, daß sich 1-Alkyl- und 1-Halogenalkyl-3-(4-chlor-6-halogenphenyl)-pyrazole bzw. -4-halogenpyrazole, die in 3-Position des Phenylrings u.a. eine geg. subst. Hydroxylgruppe tragen und die in 5-Position des Pyrazolrings durch Hydroxyl, Mercapto, niederes Alkoxy, Alkylthio, Halogenalkoxy oder Halogenalkylthio substituiert sind, zur Bekämpfung unerwünschter Pflanzen eignen.

Schließlich werden in der WO 92/06962 herbizide 4-Halogen-5-halogenalkyl-3-phenylpyrazole mit verschiedenen Substituenten am Phenylring beschrieben.

Die herbiziden Eigenschaften der bekannten Herbizide bezüglich der Schadpflanzen vermögen jedoch nur bedingt zu befriedigen.

Aufgabe der vorliegenden Erfindung war es deshalb, neue herbizid wirksame Verbindungen bereitzustellen, mit denen sich unerwünschte Pflanzen besser als bisher gezielt bekämpfen lassen.

Demgemäß wurden die vorliegenden substituierten 3-Phenylpyrazole der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Im Hinblick auf die Verwendung der substituierten 3-Phenylpyrazole I als Herbizide sind Verbindungen I bevorzugt, in denen die Variablen folgende Bedeutungen haben, und zwar jeweils für sich allein oder in Kombination:
- R³: C₁-C₄-Alkyl oder C₁-C₄-Halogenalkyl;
- R⁵: Nitro, Halogen, -COOR²⁹ oder -CO-N(R³⁰, R³¹); besonders bevorzugt ist Halogen;
- z: Sauerstoff oder Schwefel;
- X,Y: Sauerstoff oder Schwefel;
- A: eine chemische Bindung, Methylen, Ethylen oder Vinylen;
- R¹, R²⁹: unabhängig voneinander
Wasserstoff, C₁-C₈-Alkyl, C₁-C₈-Halogenalkyl, C₃-C₆-Cycloalkyl, das seinerseits ein oder zwei C₁-C₃-Alkylreste tragen kann, C₃-C₆-Alkenyl, C₃-C₆-Halogenalkenyl, Cyano-C₁-C₄-alkyl, C₃-C₆-Alkinyl, C₁-C₂-Alkoxy-C₁-C₂-alkyl, Carboxyl-C₁-C₆-alkyl, (C₁-C₄-Alkoxy)carbonyl-C₁-C₄-alkyl, Cyclopropylmethyl, C₁-C₄-Alkyl, das durch -C(R¹⁹)=N-O-(C₁-C₄-Alkyl), -C(R¹⁹)=N-O-(C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl)-phenyl substituiert ist, Phenyl, Phenyl-C₁-C₄-alkyl, Phenyl-C₂-C₄-alkenyl oder Phenoxy-C₁-C₄-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Rina-Atom einen Rest tragen kann, ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
- R³⁰, R³¹: unabhängig voneinander
Wasserstoff, C₁-C₄-Alkyl, C₁-C₄-Halogenalkyl, C₂-C₄-Alkenyl, C₂-C₄-Alkinyl, C₁-C₄-Alkoxy-C₁-C₄-alkyl, Cyano-C₁-C₄-alkyl, Carboxyl-C₁-C₄-alkyl, (C₁-C₄-Alkoxy)carbonyl-C₁-C₄-alkyl, C₃-C₆-Cycloalkyl, C₁-C₄-Alkoxy, (C₃-C₆-Cycloalkoxy)carbonyl-C₁-C₄-alkyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy und C₁-C₄-Halogenalkyl, 5-oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxy, Halogen, C₁-C₄-Alkyl, C₁-C₄-Alkoxy und C₁-C₄-Halogenalkyl;
oder
- R³⁰ und R³¹: zusammen eine Tetramethylen-, Pentamethylen- oder Ethylenoxyethylenkette, die gewünschtenfalls ein bis drei C₁-C₄-Alkylreste und/ oder einen Rest -COOR⁶ tragen kann;
- R¹⁹: Wasserstoff oder C₁-C₄-Alkyl.

Die für die Substituenten R¹ bis R³⁴ oder als Reste an (Hetero)aromaten genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Alkylcarbonyl-, Halogenalkylcarbonyl-, Alkenyl-, Alkinyl-, Halogenalkyl-, Halogenalkenyl-, Halogenalkinyl-, Cyanoalkyl-, Phenylalkyl, Carboxylalkyl-, Alkoxy-, Alkylthio-, Alkylcarbonyl-, Alkoxycarbonyl- und Alkylsulfonyl-Teile sowie der α-Alkylalkyliden-Teil können geradkettig oder verzweigt sein. Halogenierte Substituenten tragen vorzugsweise ein bis fünf gleiche oder verschiedene Halogenatome.

Im einzelnen stehen beispielsweise.
- Halogen für: Fluor, Chlor, Brom und Jod, vorzugsweise für Fluor und Chlor;
- C₁-C₆-Alkyl und die C₁-C₆-Alkyl-Teile von Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, (C₁-C₈-Alkoxy)carbonyl-C₁-C₆-alkyl, C₃-C₉-(α-Alkylalkyliden)iminooxy-C₁-C₆-alkyl, Phenyl-C₁-C₆-alkyl, Phenoxy-C₁-C₆-alkyl, Heteroaryl-C₁-C₆-alkyl, Phenyl-C₁-C₆-alkyl und Heteroaryloxy-C₁-C₆-alkyl für: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise für C₁-C₄-Alkyl, insbesondere für Methyl und Ethyl;
- C₁-C₈-Alkyl und der Alkylteil von Cyano-C₁-C₈-alkyl für: C₁-C₆-Alkyl wie vorstehend genannt, sowie z.B. für n-Heptyl, und n-Octyl, vorzugsweise für C₁-C₆-Alkyl, insbesondere für Methyl und Ethyl;
- C₁-C₄-Alkyl und die Alkylteile von C₁-C₆-Alkoxy-C₁-C₄-alkyl, C₁-C₄-Alkoxy-C₁-C₄-alkyl, Di-(C₁-C₄-alkoxy)-C₁-C₄-alkyl, C₁-C₄-Alkylthio-C₁-C₄-alkyl, (C₁-C₆-Alkoxy) carbonyl-C₁-C₄-alkyl, Di-[(C₁-C₆-Alkoxy)carbonyl]-C₁-C₄-alkyl, (C₁-C₄-Alkoxy) carbonyl-C₁-C₄-alkyl, (C₃-C₆-Cycloalkoxy) carbonyl-C₁-C₄-alkyl, C₁-C₄-Alkoxy-(C₁-C₄-alkoxy)carbonyl-C₁-C₄-alkyl, C₁-C₄-Alkylsulfonyl-C₁-C₄-alkyl, (1,3-Dioxolan-2-yl)-C₁-C₄-alkyl, (1,3-Dioxan-2-yl)-C₁-C₄-alkyl und Heteroaryl-C₁-C₄-alkyl für: Methyl, Ethyl, n-Propyl, 1-Methylethyl, n-Butyl, 1-Methylpropyl, 2-Methylpropyl und 1,1-Dimethylethyl, vorzugsweise für Methyl und Ethyl;
- C₁-C₃-Alkylreste für: Methyl, Ethyl, n-Propyl und 1-Methylethyl, vorzugsweise für Methyl;
- C₂-C₈-Alkenyl für: C₂-C₆-Alkenyl wie Ethenyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, l-Methyl-prop-2-en-1-yl, 2-Methyl-prop-2-en-1-yl, n-Penten-1-yl, n-Penten-2-yl, n-Penten-3-yl, n-Penten-4-yl, l-Methyl-but-l-en-l-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-l-en-l-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut-1-en-1-yl, 1,2-Dimethyl-but-2-en-1-yl, 1,2-Dimethyl-but-3-en-1-yl, 1,3-Dimethyl-but-l-en-l-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethylprop-2-en-1-yl, 1-Ethyl-1-me-thyl-prop-2-en-1-yl, 1-Ethyl-2-methyl-prop-1-en-1-yl und l-Ethyl-2-methyl-prop-2-en-1-yl, sowie z.B. für n-Hept-2-en-1-yl, Hept-3-en-1-yl, n-Oct-2-en-1-yl und Oct-3-en-1-yl, vorzugsweise für C₂-C₆-Alkenyl;
- C₃-C₆-Alkenyl und der Alkenyl-Teil von Hetero-aryl-C₃-C₆-alkenyl für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, l-Methyl-pent-l-en-l-yl, 2-Methyl-pent-1-en-1-yl, 3-Methyl-pent-1-en-1-yl, 4-Methyl-pent-1-en-1-yl, l-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-l-en-l-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-l-methyl-prop-2-en-1-yl, l-Ethyl-2-methyl-prop-1-en-1-yl und 1-Ethyl-2-methyl-prop-2-en-1-yl, vorzugsweise für C₃- oder C₄-Alkenyl;
- C₂-C₈-Alkinyl z.B. für: Ethinyl, Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl, und 4-Methyl-pent-2-in-5-yl, vorzugsweise für C₂-C₆-Alkinyl, insbesondere für Ethinyl und Prop-2-in-3-yl;
- C₃-C₆-Alkinyl und der Alkinyl-Teil von Heteroaryl-C₃-C₆-alkinyl für: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für C₃- oder C₄-Alkinyl, insbesondere für Ethinyl und Prop-2-in-3-yl;
- C₁-C₈-Halogenalkyl für: C₁-C₈-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für C₁-C₆-Halogenalkyl;
- C₁-C₆-Halogenalkyl für: C₁-C₆-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für C₁-C₄-Halogenalkyl, insbesondere für Trifluormethyl und 1,2-Dichlorethyl;
- C₁-C₄-Halogenalkyl für: C₁-C₄-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für Trifluormethyl und 1,2-Dichlorethyl;
- C₂-C₈-Halogenalkenyl für: C₂-C₈-Alkenyl wie vorstehend genannt, das partiell oder vollständig durch Fluor, Chlor und/ oder Brom substituiert ist, also z.B. für 2-Chlorallyl, 3-Chlorallyl und 3,3-Dichlorallyl, vorzugsweise für C₂-C₆-Halogenalkenyl;
- C₃-C₆-Halogenalkenyl für: C₃-C₆-Alkenyl wie vorstehend genannt das partiell oder vollständig durch Fluor, Chlor und/oder Brom substituiert ist, also z.B. für 2-Chlorallyl, 3-Chlorallyl und 3,3-Dichlorallyl;
- C₂-C₈-Halogenalkinyl für: C₂-C₈-Alkinyl wie vorstehend genannt, das partiell oder vollständig durch Fluor, Chlor und/ oder Brom substituiert ist, vorzugsweise für C₂-C₆-Halogen-alkenyl;
- Cyano-C₁-C₄-alkyl für: Cyanomethyl, 1-Cyanoeth-1-yl, 2-Cyano-eth-1-yl, 1-Cyanoprop-1-yl, 2-Cyanoprop-1-yl, 3-Cyano-prop-1-yl, 1-Cyanoprop-2-yl, 2-Cyanoprop-2-yl, 1-Cyano-but-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyano-but-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl, vorzugsweise für 2-Cyanoeth-1-yl;
- Cyano-C₁-C₈-alkyl für: Cyano-C₁-C₄-alkyl wie verstehend genannt, vorzugsweise für 2-Cyanoeth-1-yl;
- Phenyl-C₁-C₄-alkyl für: Benzyl, 1-Phenyleth-1-yl, 2-Phenyleth-1-yl, 1-Phenylprop-1-yl, 2-Phenylprop-1-yl, 3-Phenyl-prop-1-yl, 1-Phenylprop-2-yl, 2-Phenylprop-2-yl, 1-Phenyl-but-1-yl, 2-Phenylbut-1-yl, 3-Phenylbut-1-yl, 4-Phenyl-but-1-yl, 1-Phenylbut-2-yl, 2-Phenylbut-2-yl, 1-Phenyl-but-3-yl, 2-Phenylbut-3-yl, 1-Phenyl-2-methyl-prop-3-yl, 2-Phenyl-2-methyl-prop-3-yl, 3-Phenyl-2-methyl-prop-3-yl und 2-Benzyl-prop-2-yl;
- Carboxyl-C₁-C₄-alkyl für: Carboxylmethyl, 1-Carboxylethyl, 2-Carboxylethyl, 1-Carboxylprop-1-yl, 2-Carboxylprop-1-yl, 3-Carboxylprop-1-yl, 1-Carboxylbut-1-yl, 2-Carboxylbut-1-yl, 3-Carboxylbut-1-yl, 4-Carboxylbut-1-yl, 1-Carboxylbut-2-yl, 2-Carboxylbut-2-yl, 3-Carboxylbut-2-yl, 3-Carboxylbut-2-yl, 4-Carboxylbut-2-yl, 1-(Carboxylmethyl)-eth-1-yl, 1-(Carboxyl-methyl)-1-(methyl)-eth-1-yl und 1-(Carboxylmethyl)-prop-1-yl, vorzugsweise für Carboxylmethyl und 2-Carboxylethyl;
- Carboxyl-C₁-C₆-alkyl für: Carboxyl-C₁-C₄-alkyl wie vorstehend genannt, sowie für 5-Carboxylpent-1-yl, vorzugsweise für Carboxyl-C₁-C₄-alkyl;
- C₁-C₄-Alkoxy und die Alkoxy-Teile von C₁-C₄-Alkoxy-C₁-C₄-alkyl, Di-(C₁-C₄-alkoxy)-C₁-C₄-alkyl, (C₁-C₄-Alkoxy) carbonyl-C₁-C₄-alkyl, C₁-C₄-Alkoxy-(C₁-C₄-alkoxy)carbonyl-C₁-C₆-alkyl, C₁-C₄-Alkoxy-(C₁-C₄-alkoxy)carbonyl-C₁-C₄-alkyl und C₁-C₄-Alkoxy-C₁-C₄-alkoxy für: Methoxy, Ethoxy, n-Propoxy, 1-Methylethoxy, n-Butoxy, 1-Methyl-propoxy, 2-Methylpropoxy und 1,1-Dimethylethoxy, vorzugsweise für Methoxy, Ethoxy und 1-Methylethoxy;
- C₁-C₆-Alkoxy und der Alkoxy-Teil von C₁-C₆-Alkoxy-C₁-C₄-alkyl für: C₁-C₄-Alkoxy wie vorstehend genannt, sowie für n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, vorzugsweise für Methoxy, Ethoxy und 1-Methylethoxy;
- C₁-C₈-Alkoxy für: C₁-C₆-Alkoxy wie vorstehend genannt, sowie z.B. für n-Heptoxy und n-Octoxy, vorzugsweise für C₁-C₆-Alkoxy, insbesondere für Methoxy, Ethoxy und 1-Methylethoxy;
- C₁-C₄-Alkylthio und der Alkylthio-Teil von C₁-C₄-Alkylthio-C₁-C₄-alkyl für: Methylthio, Ethylthio, n-Propylthio, 1-Methylethylthio, n-Butylthio, 1-Methylpropylthio, 2-Methylpropylthio und 1,1-Dimethylethylthio, vorzugsweise für Methylthio, Ethylthio und 1-Methylethylthio;
- C₁-C₆-Alkylthio für: C₁-C₄-Alkylthio wie vorstehend genannt, sowie für n-Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, n-Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise für Methylthio, Ethylthio und 1-Methylethylthio;
- (C₁-C₄-Alkyl)carbonyl für: Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl und 1,1-Dimethylethylcarbonyl, vorzugsweise für Methylcarbonyl, Ethylcarbonyl und n-Propylcarbonyl;
- (C₁-C₄-Halogenalkyl)carbonyl für: C₁-C₄-Alkyl wie vorstehend genannt, das partiell oder vollständig durch Fluor, Chlor und/oder Brom substituiert ist, also z.B. Chlormethylcarbonyl, Dichlormethylcarbonyl, Trichlormethylcarbonyl, Fluormethylcarbonyl, Difluormethylcarbonyl, Trifluormethylcarbonyl, Chlorfluormethylcarbonyl, Dichlorfluormethylcarbonyl, Chlordifluormethylcarbonyl, 1-Fluorethylcarbonyl, 2-Fluorethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 3-Chlorpropylcarbonyl und Heptafluorpropylcarbonyl, vorzugsweise für Trifluormethylcarbonyl und 1,2-Dichlorethylcarbonyl;
- (C₁-C₆-Alkoxy)carbonyl und der Alkoxycarbonyl-Teil von (C₁-C₆-Alkoxy)carbonyl-C₁-C₄-alkyl für: Methoxycarbonyl, Ethoxycarbonyl, n-Propoxycarbonyl, 1-Methyl-ethoxycarbonyl, n-Butoxycarbonyl, 1-Methylpropoxycarbonyl, 2-Methylpropoxycarbonyl, 1,1-Dimethylethoxycarbonyl, n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl und 1-Ethyl-2-methyl-propoxycarbonyl, vorzugsweise für (C₁-C₄-Alkoxy)carbonyl, insbesondere für Methoxycarbonyl, Ethoxycarbonyl und 1-Methylethoxycarbonyl;
- der Alkoxycarbonyl-Teil von (C₁-C₈-Alkoxy)carbonyl-C₁-C₆-alkyl für: (C₁-C₆-Alkoxy)carbonyl wie vorstehend genannt, sowie z.B. für n-Heptoxycarbonyl und n-Octoxycarbonyl, vorzugsweise für (C₁-C₆-Alkoxy)carbonyl, insbesondere für Methoxycarbonyl, Ethoxycarbonyl und 1-Methylethoxycarbonyl;
- der Alkylsulfonylteil von C₁-C₄-Alkylsulfonyl-C₁-C₄-alkyl für: Methylsulfonyl, Ethylsulfonyl, n-Propylsulfonyl, 1-Methylethylsulfonyl, n-Butylsulfonyl, 1-Methyl-propylsulfonyl, 2-Methylpropylsulfonyl und 1,1-Dimethylethylsulfonyl, vorzugsweise für Methylsulfonyl und Ethylsulfonyl;
- der C₃-C₉-(α-Alkylalkyliden)-Teil von C₃-C₉-(α-Alkylalkyliden)iminooxy-C₁-C₆-alkyl z.B. für: α-Methylethyliden, α-Methylpropyliden und α-Ethylpropyliden, insbesondere für α-Methylethyliden;
- C₃-C₆-Cycloalkyl für: Cyclopropyl, Cyclobutyl, Cyclopentyl und Cyclohexyl, vorzugsweise für Cyclopropyl und Cyclopentyl;
- C₃-C₇-Cycloalkyl für: C₃-C₆-Cycloalkyl wie vorstehend genannt, sowie für Cycloheptyl, vorzugsweise für Cyclopropyl und Cyclopentyl;
- C₃-C₈-Cycloalkyl für: C₃-C₆-Cycloalkyl wie vorstehend genannt, sowie für Cycloheptyl und Cyclooctyl, vorzugsweise für Cyclopropyl, Cyclopentyl und Cyclohexyl;
- C₅-C₇-Cycloalkenyl für: Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, vorzugsweise für Cyclopent-1-enyl;
- der Cycloalkoxycarbonyl-Teil von (C₃-C₆-Cycloalkoxy)carbonyl-C₁-C₄-alkyl für: Cyclopropoxycarbonyl, Cyclobutoxycarbonyl, Cyclopentoxycarbonyl und Cyclohexoxycarbonyl, vorzugsweise für Cyclopropoxycarbonyl und Cyclopentoxycarbonyl;
- 5- oder 6-gliedriges Heteroaryl und der Heteroaryl-Teil von Heteroaryl-C₁-C₄-alkyl für: z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise für 3-Pyrazolyl, 2-Pyridinyl und 2-Thienyl.

Die 3-Phenylpyrazol-Derivate der Formel I sind auf verschiedene Weise erhältlich, vorzugsweise nach einem der folgenden Verfahren:
A) Umsetzung eines β-Ketocarbonsäurederivats III mit Hydrazin oder einem Hydrazin-Derivat in einem inerten Lösungsmittel (vgl. z.B. JP-A 04/225 937 und JP-A 03/072 460) und Alkylierung des Verfahrensproduktes IV: 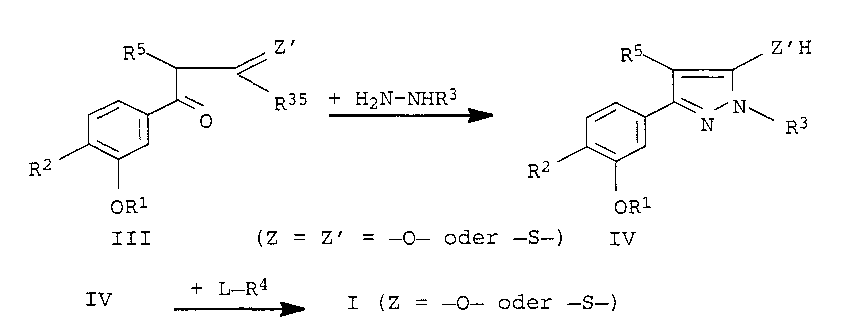
   - L: steht für eine geeignete Abgangsgruppe wie Halogen, -O-SO₂CH₃, -O-SO₂CF₃, -O-SO₂C₄F₉ oder -O-SO₂(p-CH₃-C₆H₄);
   - R³⁵: steht vorzugsweise für C₁-C₄-Alkoxy, C₁-C₄-Alkylcarbonyloxy oder Halogen.

   Das Lösungsmittel kann aprotisch oder protisch sein. Geeignet sind z.B. organische Säuren wie Essigsäure, Kohlenwasserstoffe, halogenierte Kohlenwasserstoffe, Ether wie Ethylenglykoldimethylether, Alkohole wie Methanol und Ethanol, sowie Sulfoxide.
   Die Reaktionstemperatur wird hauptsächlich vom Schmelzpunkt der Verbindung III und dem Siedepunkt des Reaktionsgemisches vorgegeben. Bevorzugt arbeitet man bei etwa 60 bis 120°C.
   Im allgemeinen setzt man etwa die 0,95- bis 5-fache molare Menge, zweckmäßigerweise die 1- bis 1,4-fache Menge, an Hydrazin oder Hydrazin-Derivat, bezogen auf den β-Ketocarbonsäurederivats III, ein.
   Die Menge an Alkylierungsmittel L-R⁴ liegt üblicherweise ebenfalls bei der 0,95- bis zur 5-fachen molaren Menge, bezogen auf das Zwischenprodukt IV.
   Mit Blick auf die bevorzugten Reste R³ an den 3-Phenylpyrazolen I sind unter den Hydrazin-Derivaten diejenigen besonders bevorzugt, die eine Alkylgruppe tragen.
   Die Alkylierung erfolgt normalerweise mit dem Halogenid, vorzugsweise dem Chlorid oder Bromid, oder mit dem Sulfat eines Alkans oder Halogenalkans, gewünschtenfalls in Gegenwart einer organischen Base, z.B. einem Trialkylamin oder Pyridin, oder einer anorganischen Base, z.B. einem Alkalimetallcarbonat.
   Zweckmäßigerweise wir die Alkylierung in einem inerten organischen Lösungsmittel vorgenommen, z.B. in einem aliphatischen oder cyclischen Ether wie 1,2-Dimethoxyethan, Tetrahydrofuran und Dioxan, in einem aliphatischen Keton wie Aceton, in einem Amid wie Dimethylformamid, in einem Sulfoxid wie Dimethylsulfoxid oder in einer Mischung aus einem dieser Lösungsmittel und Wasser.
   Die Reaktion ist im allgemeinen bei einer Temperatur von 0°C bis zur Siedetemperatur des Reaktionsgemisches ausführbar. Vorzugsweise arbeitet man bei etwa 20 bis 80°C.
   Im Falle Z = S kann das Produkt zum Sulfoxid oder Sulfon auf an sich bekannte Weise oxidiert werden (vgl. z.B. Houben-Weyl, Methoden der Organischen Chemie, Bd. 9, Georg Thieme Verlag, Stuttgart, 4. Auflage 1955, S. 211ff und 227ff; Org. Synth. Coll. Vol. V, 791): 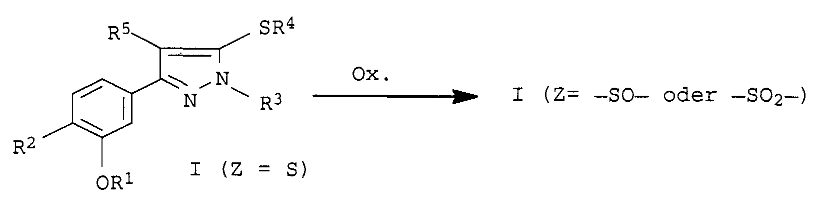
B) Halogenierung von Verbindungen I mit R⁵ = Wasserstoff: 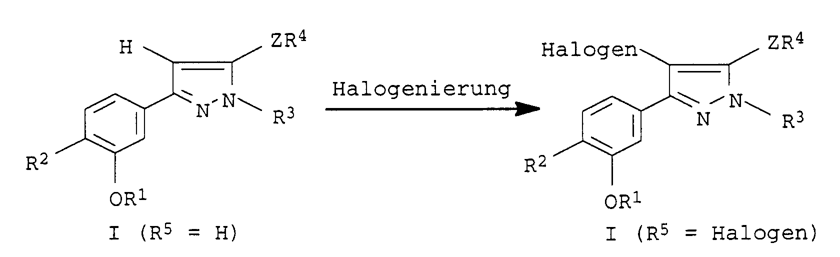
   Die Umsetzung kann in einem inerten Lösungs- oder Verdünnungsmittel oder lösungsmittelfrei vorgenommen werden.
   Beispiele für geeingete Lösungsmittel sind organische Säuren, anorganische Säuren, Kohlenwasserstoffe, halogenierte Kohlenwasserstoffe, aromatische Kohlenwasserstoffe, Ether, Sulfide, Sulfoxide und Sulfone.
   Als Halogenierungsmittel kommen beispielsweise Chlor, Brom, N-Bromsuccinimide, N-Chlorsuccinimide oder Sulfurylchlorid in Betracht. Je nach Ausgangsverbindung und Halogenierungsmittel kann der Zusatz eines Radikalstarters, beispielsweise eines organischen Peroxides wie Dibenzoylperoxid oder einer Azoverbindung wie Azobisisobutyronitril, oder Bestrahlung mit Licht vorteilhaft auf den Reaktionsverlauf wirken.
   Die Menge an Halogenierungsmittel ist nicht kritisch. Sowohl unterstöchiometrische Mengen als auch große Überschüsse an Halogenierungsmittel, bezogen auf die zu halogenierende Verbindung I mit R⁵ = Wasserstoff, sind möglich.
   Bei Verwendung eines Radikalstarters ist in der Regel eine katalytische Menge ausreichend.
   Die Reaktionstemperatur liegt normalerweise bei (- 100) bis 200° C, vornehmlich bei 10 bis 100° C oder dem Siedepunkt des Reaktionsgemisches.
C) Nitrierung von Verbindungen V, Reduktion der Nitrogruppe und Überführung der resultierenden Aniline VII in die Wirkstoffe I: 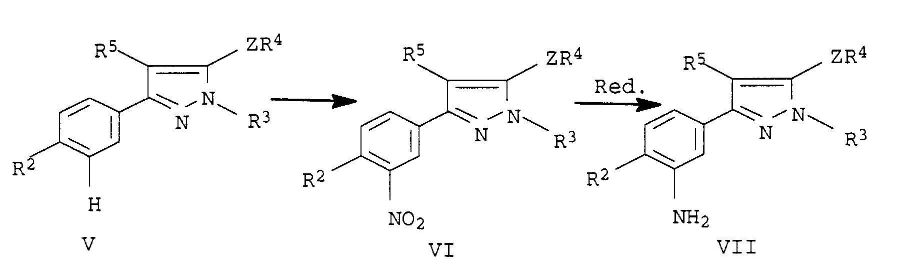
   Als Nitrierungs-Reagenzien kommen beispielsweise Salpetersäure in unterschiedlicher Konzentration, auch konzentrierte und rauchende Salpetersäure, Mischungen von Schwefelsäure und Salpetersäure, Acetylnitrate und Alkylnitrate in Betracht.
   Die Reaktion kann entweder lösungsmittelfrei in einem Überschuß des Nitrierungs-Reagenzes oder in einem inerten Lösungs- oder Verdünnungsmittel durchgeführt werden, wobei z.B. Wasser, Mineralsäuren, organische Säuren, Chlorkohlenwasserstoffe wie Methylenchlorid, Anhydride wie Essigsäureanhydrid und Mischungen dieser Solventien geeignet sind.
   Die Ausgangsverbindung V und das Nitrierungs-Reagenz werden zweckmäßigerweise in etwa äquimolaren Mengen eingesetzt; zur Optimierung des Umsatzes an zu nitrierender Verbindung kann es jedoch vorteilhaft sein, das Nitrierungs-Reagenz im Überschuß zu verwenden, bis etwa zur 10-fachen molaren Menge. Bei der Reaktionsführung ohne Lösungsmittel im Nitrierungs-Reagenz liegt dieses in einem noch größeren Überschuß vor.
   Die Reaktionstemperatur liegt normalerweise bei (-100) bis 200°C, bevorzugt bei (-30) bis 50°C.
   Die Aufarbeitung des Reaktionsgemisches kann auf bekannte Weise erfolgen, beispielsweise durch Verdünnen der Reaktionslösung mit Wasser und anschließender Isolierung des Produktes mittels Filtration, Kristallisation oder Lösungsmittelextraktion.
C1) Reduktion mit einem Metall wie Eisen, Zink oder Zinn unter sauren Reaktionsbedingungen oder mittels komplexen Hydriden wie Lithiumaluminiumhydrid und Natriumborhydrid:
   Das Lösungsmittel, z.B. Wasser, ein Alkohol wie Methanol, Ethanol und Isopropanol oder ein Ether wie Diethylether, Methyl-tert.-butylether, Dioxan, Tetrahydrofuran und Ethylenglykoldimethylether, ist abhängig vom gewählten Reduktionsmittel.
   Bei der Reduktion mit einem Metall arbeitet man vorzugsweise lösungsmittelfrei in einer anorganische Säure, insbesondere konzentrierter oder verdünnter Salzsäure, oder einer organischen Säure wie Essigsäure. Es ist aber auch möglich, der Säure ein inertes Lösungsmittel wie vorstehend genannt zuzumischen.
   Die Ausgangsverbindung VI und das Reduktionsmittel werden zweckmäßigerweise in etwa äquimolaren Mengen eingesetzt; zur Optimierung des Reaktionsverlaufes kann es jedoch vorteilhaft sein, eine der beiden Komponenten im Überschuß zu verwenden, bis etwa zur 10-fachen molaren Menge.
   Die Menge an Säure ist nicht kritisch. Um die Ausgangsverbindung möglichst vollständig zu reduzieren verwendet man zweckmäßigerweise mindestens eine äquivalente Menge an Säure.
   Die Reaktionstemperatur liegt im allgemeinen bei (-30) bis 200°C, bevorzugt bei 0 bis 80°C.
   Nach Beendigung der Reaktion wird die Reaktionsmischung üblicherweise mit Wasser verdünnt und das Produkt durch Filtration, Kristallisation oder Extraktion mit einem Lösungsmittel, das mit Wasser weitgehend unmischbar ist, z.B. mit Essigsäureethylester, Diethylether oder Methylenchlorid, isoliert. Gewünschtenfalls kann das Produkt anschließend auf bekannte Weise gereinigt werden.
C2) Katalytische Hydrierung mit Wasserstoff:
   Geeignete Katalysatoren sind beispielsweise Raney-Nickel, Palladium auf Kohle, Palladiumoxid, Platin und Platinoxid, wobei im allgemeinen eine Katalysatormenge von 0,05 bis 10,0 mol-%, bezogen auf die zu reduzierende Verbindung, ausreichend ist.
   Man arbeitet entweder lösungsmittelfrei oder in einem inerten Lösungs- oder Verdünnungsmittel, z.B. in Essigsäure, einem Gemisch aus Essigsäure und Wasser, Essigsäureethylester, Ethanol oder in Toluol.
   Nach Abtrennen des Katalysators kann die Reaktionslösung wie üblich auf das Produkt hin aufgearbeitet werden.
   Die Hydrierung kann bei Normaldruck oder unter erhöhtem Druck durchgeführt werden.
   Die Verbindungen VII können auf an sich bekannte Weise diazotiert (siehe hierzu z.B. Houben-Weyl, Methoden der Organischen Chemie, Band E16d, Teil 2, Georg Thieme Verlag, Stuttgart, 4. Auflage 1992, Seiten 1241 bis 1314 sowie die dort zitierte Literatur) und analog der in Org. Synth. Coll. Vol. 3, 130 (1955) beschriebenen Phenolverkochung in die Verbindungen I übergeführt werden.
D) Nucleophile Cyanid-Substitution von Verbindungen VI mit R² = F und Nucleophile Alkoholatsubstitution der Verfahrensprodukte VI mit R² = CN auf an sich bekannte Weise (siehe z.B. Houben-Weyl, Methoden der Organischen Chemie, Bd. 6/3, Georg Thieme Verlag, Stuttgart, 4. Auflage 1965, S. 75ff.); auf diese Weise sind bevorzugt Verbindungen I mit R² = CN und R⁶ = Niederalkyl herstellbar: 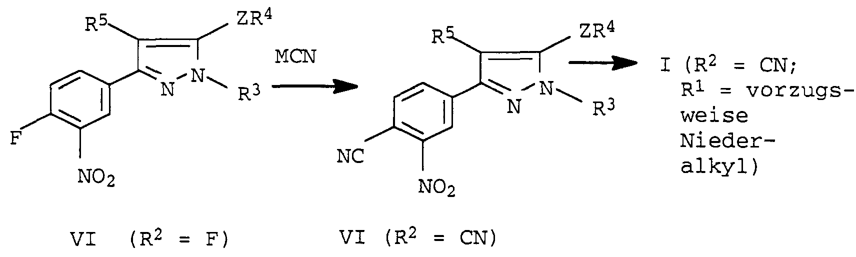
   M steht für ein metallisches oder organisches Kation, vorzugsweise für ein Alkalimetall- oder Tetraalkylammoniumion.
   Die Cyanid-Substitituion wird üblicherweise in einem polaren aprotischen Lösungsmittel wie Dimethylsulfoxid, N,N-Dimethylformamid und Sulfolan vorgenommen, wobei die Reaktionstemperatur zwischen dessen Schmelz- und Siedepunkt, insbesondere bei 0 bis 100°C liegt.
   Vorzugsweise kommt ein geringfügiger molarer Überschuß des Cyanids MCN zur Anwendung. Zur Optimierung des Umsatzes kann es jedoch vorteilhaft sein, einen großen Überschuß an MCN zu verwenden, bis etwa zur fünffachen molaren Menge, bezogen auf die Menge an Ausgangsverbindung VI mit R² = Fluor.
   Die Aufarbeitung des Reaktionsgemisches kann auf bekannte Weise erfolgen, etwa durch Verdünnen des Reaktionsgemisches mit Wasser und nachfolgender Isolierung des Produkts mittels Filtration, Kirstallisation oder Lösungsmittelextraktion.
   Die Verfahrensprodukte I (R¹ ≠ H; R² = CN) können gewünschtenfalls auf an sich bekannte Weise zu Verbindungen I mit R¹ = H (R² = CN) gespalten werden (vgl. z.B. Houben-Weyl, Methoden der Organischen Chemie, Bd. 6/1c, Georg Thieme Verlag Stuttgart, 4. Auflage 1976, S. 313ff.).
   Sofern nicht anders angegeben werden die vorstehend beschriebenen Reaktionen zweckmäßigerweise bei Atmosphärendruck oder unter dem Eigendruck des jeweiligen Reaktionsgemisches vorgenommen.
   Die substituierten 3-Phenylpyrazole I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die reinen Isomeren getrennt werden können. Reine optisch aktive Isomere lassen sich vorteilhaft aus entsprechenden optisch aktiven Ausgangsprodukten herstellen.

Salze landwirtschaftlich brauchbarer Kationen der substituierten 3-Phenylpyrazole I können durch Behandeln der freien Verbindungen I z.B.
- mit Natrium- oder Kaliumhydroxid in wäßriger Lösung oder in einem organischen Lösungsmittel wie Methanol, Ethanol, Aceton und Toluol oder
- mit Natriumhydrid in einem organischen Lösungsmittel wie Dimethylformamid
erhalten werden.

Salze substituierter 3-Phenylpyrazole I, bei denen das Kation nicht zu der Gruppe der Alkalimetalle gehört, können üblicherweise durch Umsalzen der entsprechenden Alkalimetallsalze hergestellt werden.

Diejenigen Verbindungen I, die die funktionelle Gruppe >C=N- enthalten, können durch Reaktion mit der entsprechenden Säure in ihre Säureadditionssalze übergeführt werden.

Die Säureaddition kann in wäßriger Lösung oder in einem organischen Lösungsmittel wie Methanol, Ethanol, Aceton, Toluol und Ether durchgeführt werden. Auch die Säureadditionssalze von I können umgesalzt werden, wodurch landwirtschaftlich brauchbare Salze mit weiteren Anionen zugänglich sind.

Die Salzbildungen verlaufen normalerweise bereits bei etwa 20°C mit ausreichender Geschwindigkeit.

Die Isolierung der Salze kann z.B. durch Fällen mit einem geeigneten inerten Lösungsmittel oder durch Abdampfen des Lösungsmittels erfolgen.

Die substituierten 3-Phenylpyrazole I und deren Salze eignen sich, sowohl als Isomerengemische als auch in Form der reinen Isomeren, als Herbizide. Sie können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I in Kulturen, die durch Züchtung und/oder gentechnische Methoden gegen die Wirkung von I weitgehend resistent sind, eingesetzt werden.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden, etwa zwischen 0,01 und 95 Gew. %, vorzugsweise zwischen 0,5 und 90 Gew.%. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
- I.: 20 Gewichtsteile einer Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
- II.: 20 Gewichtsteile einer Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- III.: 20 Gewichtsteile eines Wirkstoffs I werden in einer Mischung gelöst, die aus 25 Gewichtsteilen Cyclohexanon, 65 Gewichtsteilen einer Mineralölfraktion vom Siedepunkt 210 bis 280⁰C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- IV.: 20 Gewichtsteile eines Wirkstoffs I werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
- V.: 3 Gewichtsteile eines Wirkstoffs I werden mit 97 Gewichtsteilen feinteiligem Kaolin vermischt. Man erhält auf diese Weise ein Stäubemittel, das 3 Gew.% des Wirkstoffs enthält.
- VI.: 20 Gewichtsteile eines Wirkstoffs I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten 3-Phenylpyrazole I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1- Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

4-Chlor-3-(4-cyano-3-methoxyphenyl)-5-difluor-methoxy-1-methyl-1H-pyrazol (Nr. If.001))

Zu einer Lösung von 1,4 g (4,3 mmol) 4-Chlor-3-(4-cyano-3-nitrophenyl)-5-difluormethoxy-1-methyl-1H-pyrazol in 30 ml Methanol wurden 0,9 ml einer 30 gew.-%igen Lösung von Natriummethylat in Methanol gegeben. Nach 6 Stunden Rühren bei Raumtemperatur kühlte man ca. 16 Std. auf 0°C. Danach wurde das feste Reaktionsprodukt abgetrennt. Ausbeute: 0,6 g.

In den folgenden Tabellen 1 - 7 sind weitere Verbindungen angegeben, die auf die gleiche Weise hergestellt wurden oder entweder nach den vorstehend beschriebenen Verfahren oder nach an sich bekannten Methoden herstellbar sind.

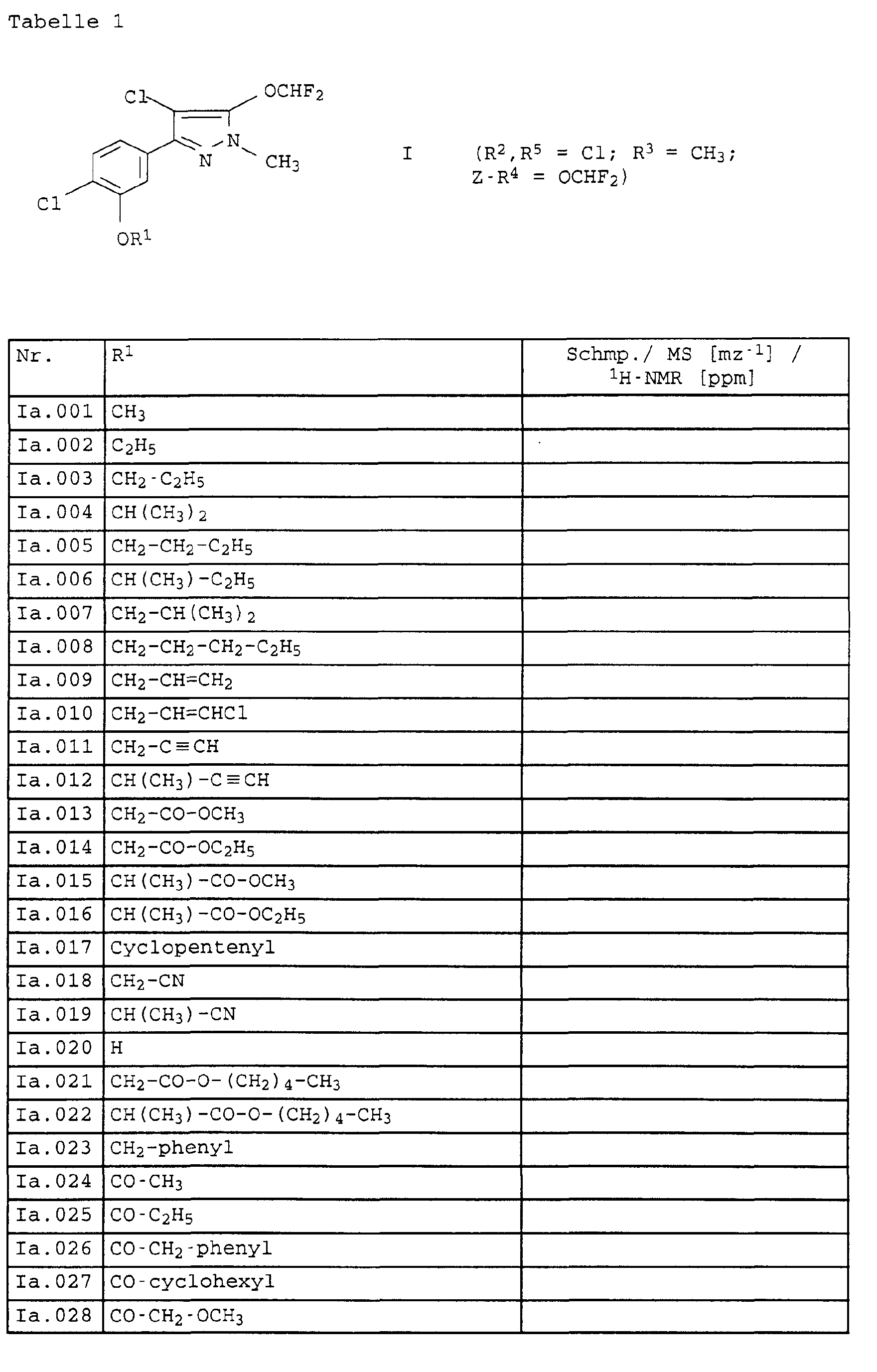

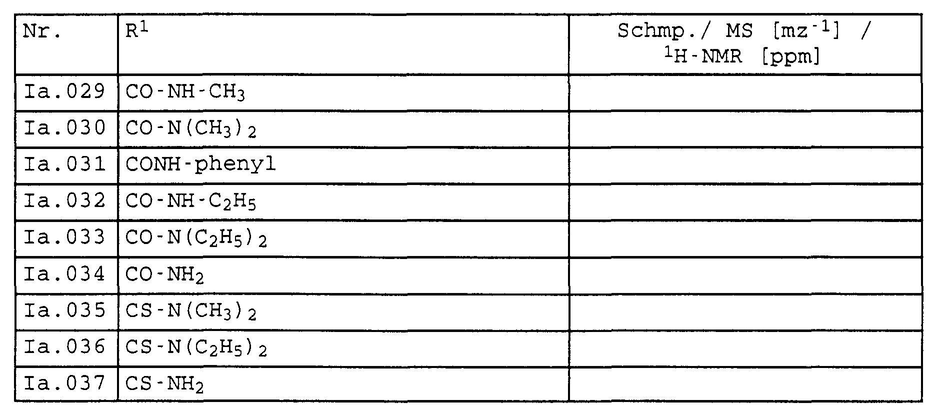

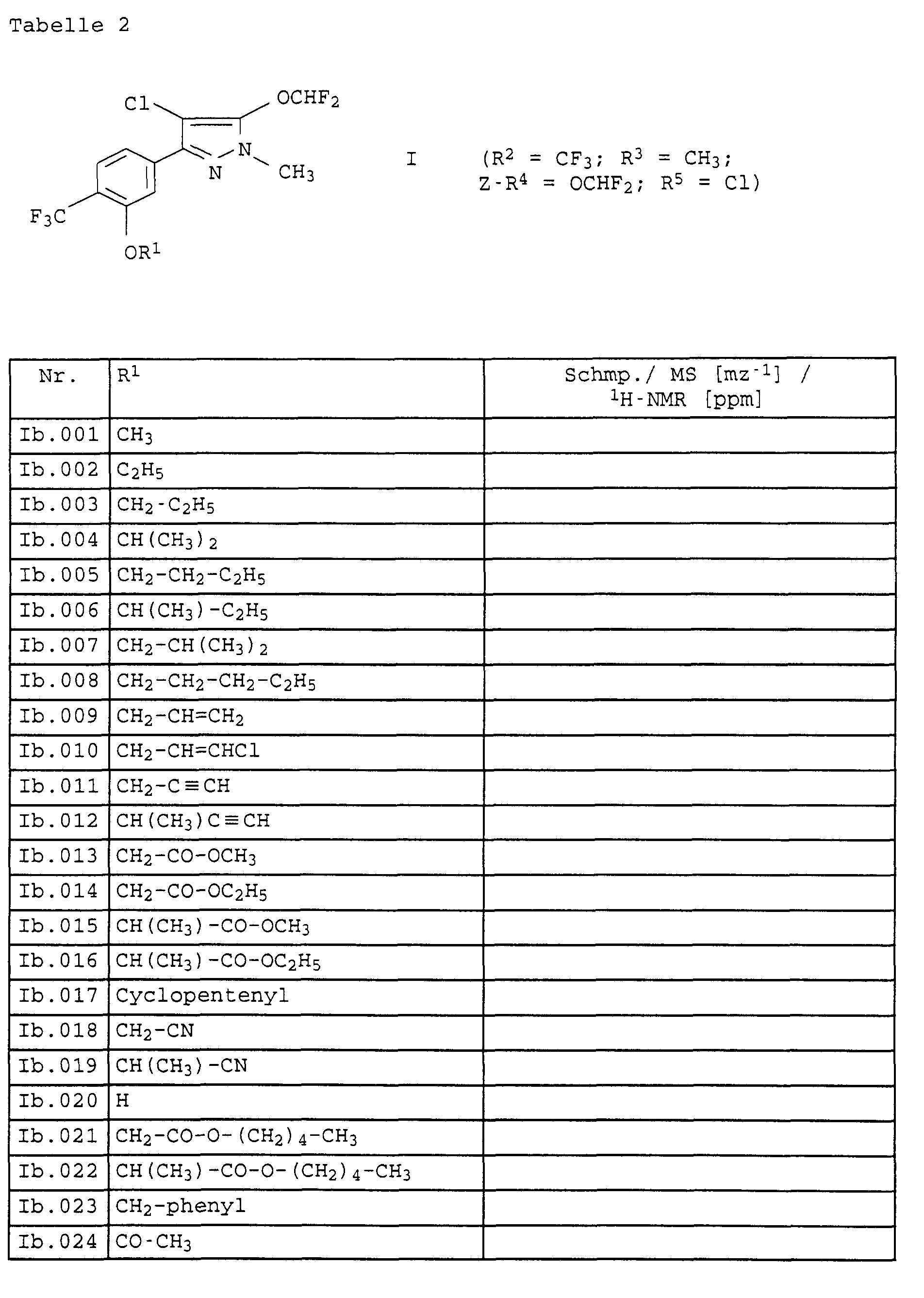

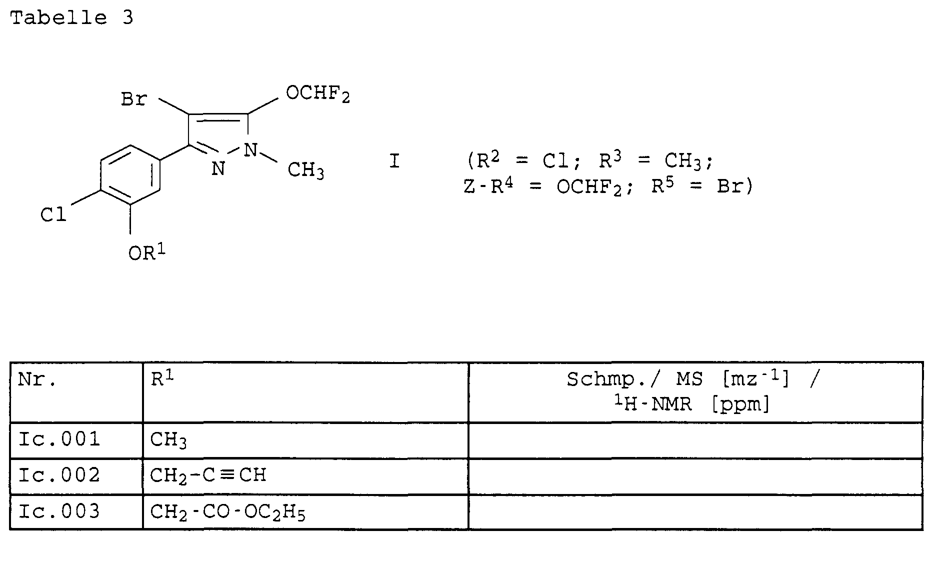

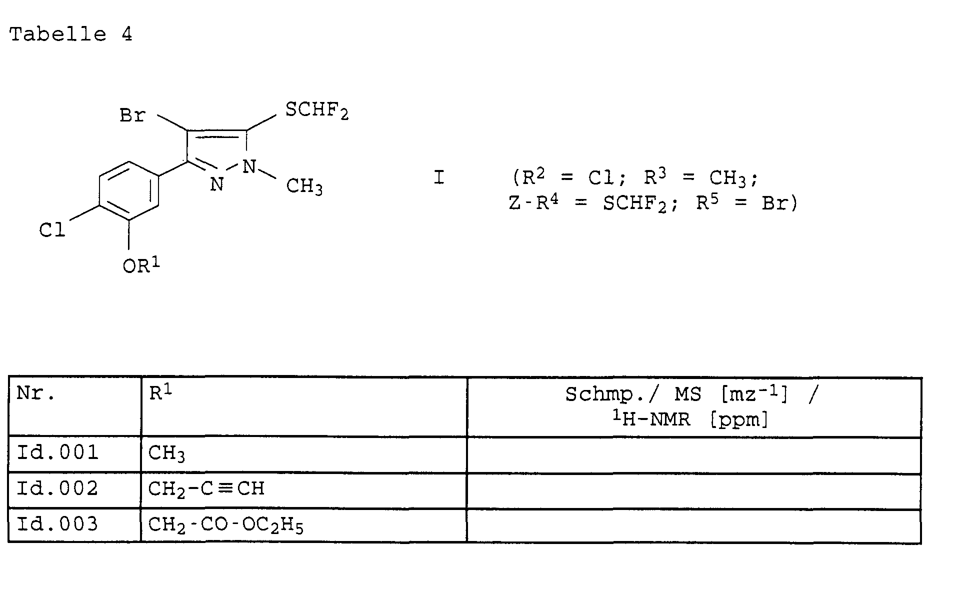

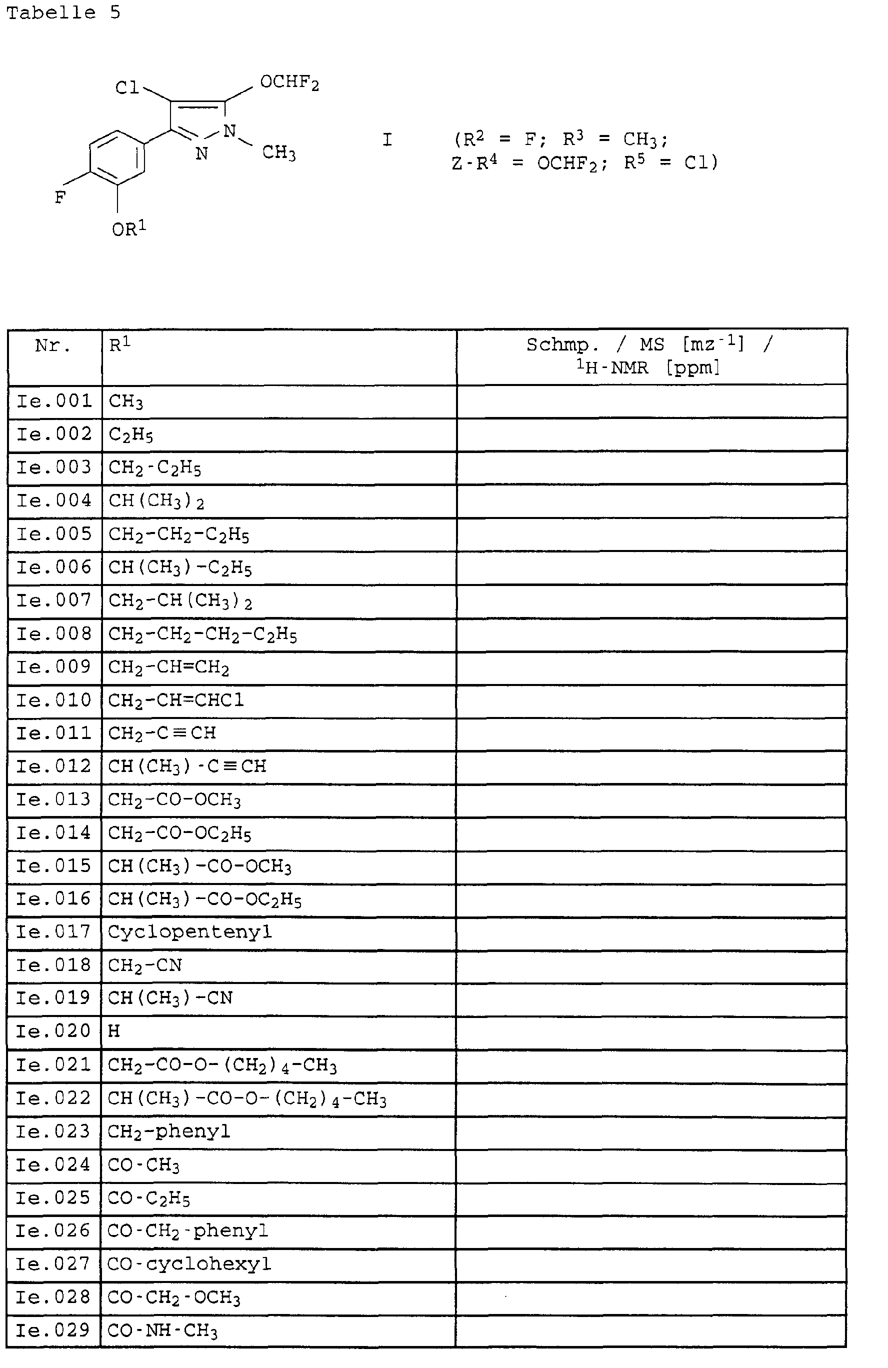

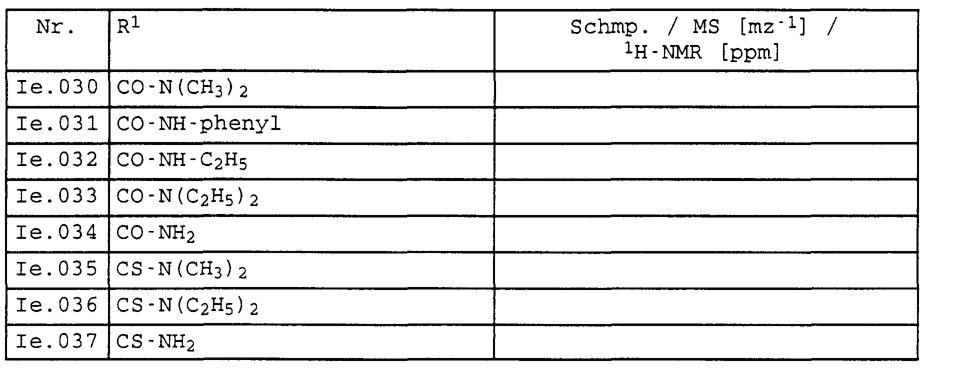

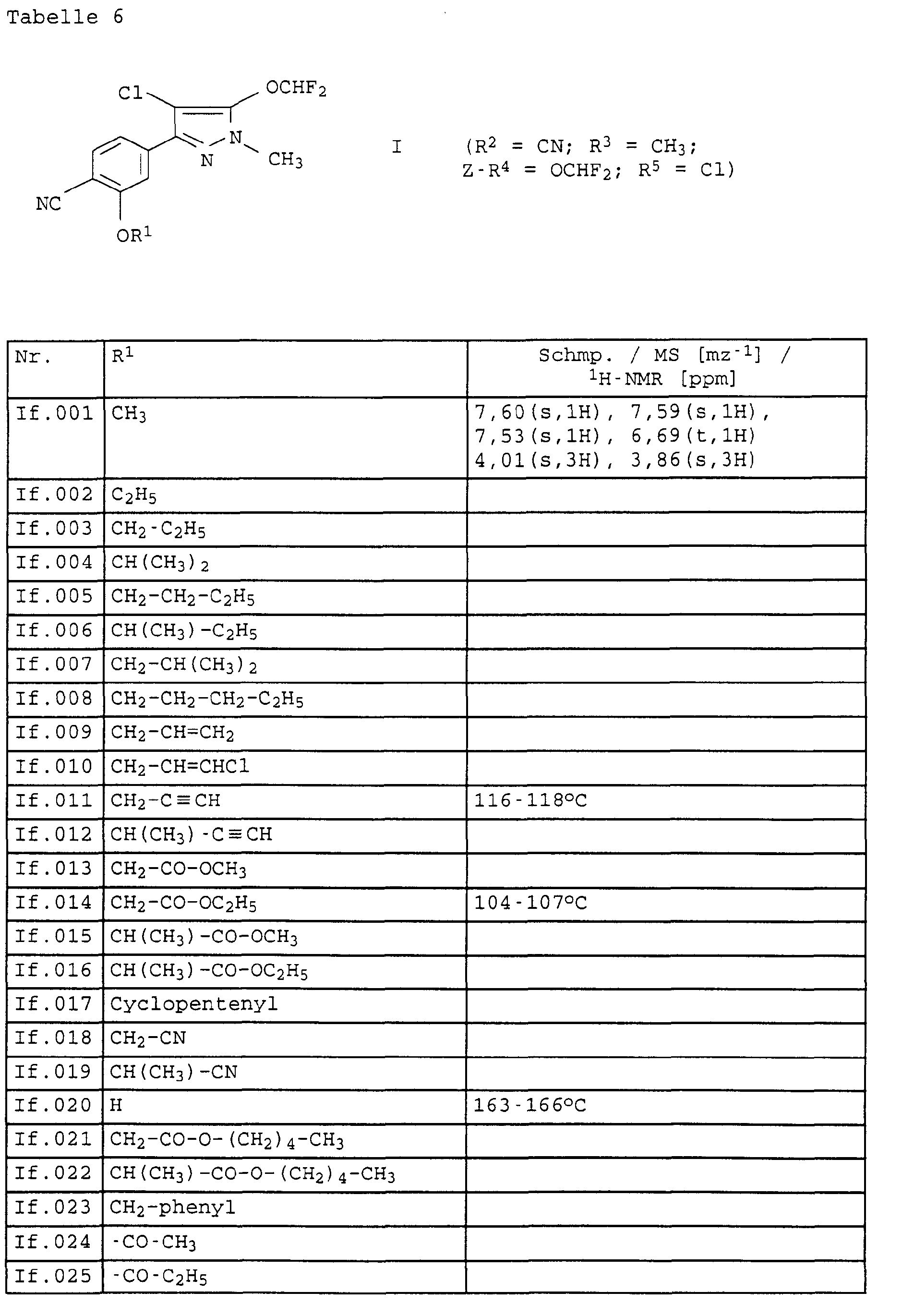

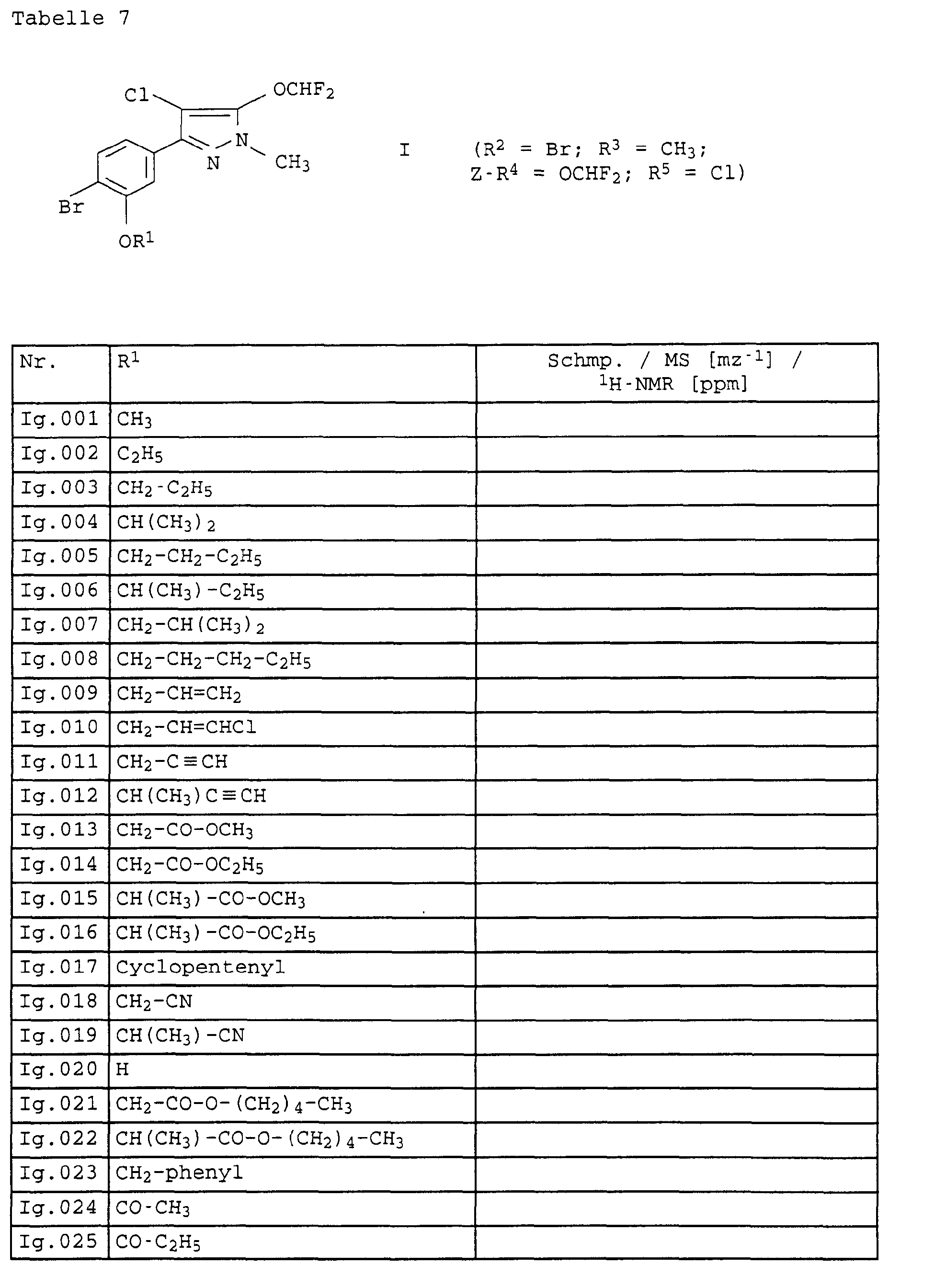

### Anwendungsbeispiele

Die herbizide Wirkung der substituierten 3-Phenylpyrazole der Formel I ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 - 25 ⁰C bzw. 20 - 35 ⁰C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. Substituierte 3-Phenylpyrazole der Formel I 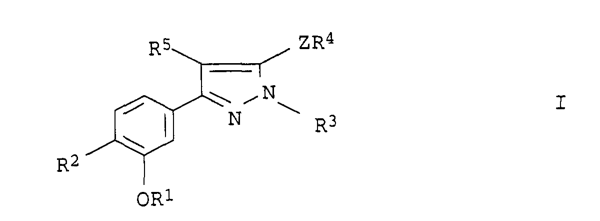 in der die Variablen folgende Bedeutung haben:
R² Cyano, Trifluormethyl oder Halogen;
R³ Wasserstoff, C₁-C₄-Alkyl oder C₁-C₄-Halogenalkyl;
R⁴ C₁-C₄-Alkyl oder C₁-C₄-Halogenalkyl;
R⁵ Wasserstoff, Nitro, Halogen, -COOR²⁹ oder -CO-N(R³⁰,R³¹);
Z Sauerstoff, Schwefel, -SO- oder -SO₂-;
X,Y unabhängig voneinander Sauerstoff oder Schwefel;
A eine chemische Bindung, Methylen, Ethylen, 1,3-Propylen, 1,4-Butylen, Vinylen oder 1,4-Butadienylen;
R¹, R²⁹ unabhängig voneinander
Wasserstoff, C₁-C₈-Alkyl, C₁-C₈-Halogenalkyl, C₃-C₇-Cycloalkyl, das seinerseits ein bis drei C₁-C₃-Alkylreste tragen kann, C₃-C₆-Alkenyl, C₅-C₇-Cycloalkenyl, das seinerseits ein bis drei C₁-C₃-Alkylreste tragen kann, C₃-C₆-Halogenalkenyl, Cyano-C₁-C₈-alkyl, C₃-C₆-Alkinyl, C₁-C₄-Alkoxy-C₁-C₄-alkyl, 2-Tetrahydrofuranyl-C₁-C₈-alkyl, 3-Oxetanyl, 3-Thiethanyl, Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, Cyclopropylmethyl, (1-Methylthiocycloprop-1-yl)methyl, C₃-C₉-(α-Alkylalkyliden)iminooxy-C₁-C₆-alkyl, (C₁-C₄-Alkyl)carbonyl, C₁-C₄-Alkyl, das durch -C(R¹⁹)=N-O-(C₁-C₄-Alkyl), -C(R¹⁹)=N-O-(C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl) -R³⁴ substituiert ist, Phenyl, Phenyl-C₁-C₆-alkyl, Phenyl-C₂-C₆-alkenyl, Phenyl-C₃-C₆-alkinyl oder Phenoxy-C₁-C₆-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl, Heteroaryl-C₁-C₆-alkyl, Heteroaryl-C₃-C₆-alkenyl, Heteroaryl-C₃-C₆-alkinyl oder Heteroaryloxy-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ringglied einen Rest tragen kann, jeweils ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
R³⁰, R³¹ unabhängig voneinander
Wasserstoff, C₁-C₈-Alkyl, C₁-C₈-Halogenalkyl, C₂-C₈-Alkenyl, C₂-C₈-Alkinyl, C₁-C₄-Alkoxy-C₁-C₄-alkyl, C₁-C₄-Alkylthio-C₁-C₄-alkyl, Cyano-C₁-C₈-alkyl, Carboxyl-C₁-C₄-alkyl, (C₁-C₄-Alkoxy)carbonyl-C₁-C₄-alkyl, C₁-C₄-Alkylsulfonyl-C₁-C₄-Alkyl, C₃-C₈-Cycloalkyl, C₁-C₆-Alkoxy, (C₃-C₆-Cycloalkoxy)carbonyl-C₁-C₄-alkyl, C₁-C₄-Alkoxy-(C₁-C₄-alkoxy)carbonyl-C₁-C₄-alkyl, (C₁-C₄-Alkyl)carbonyl, (C₁-C₄-Halogenalkyl)carbonyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
oder
R³⁰ und R³¹ zusammen eine Tetramethylen-, Pentamethylen- oder Ethylenoxyethylenkette, die gewünschtenfalls ein bis drei C₁-C₄-Alkylreste und/oder einen Rest -COOR⁶ tragen kann;
R¹⁹ Wasserstoff, C₁-C₄-Alkyl, Phenyl oder Benzyl;
R³⁴ Phenyl oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei jeder Phenyl- oder Heteroarylring unsubstituiert sein oder an jedem substituierbaren Ringglied gewünschtenfalls einen Substituenten tragen kann, jeweils ausgewählt aus der Gruppe bestehend aus Hydroxy, Nitro, Cyano, Halogen, C₁-C₄-Alkyl, C₁-C₄-Halogenalkyl, C₁-C₄-Alkoxy und C₁-C₄-Alkylthio,
sowie die landwirtschaftlich brauchbaren Salze von I.

2. Verwendung der substituierten 3-Phenylpyrazole I gemäß Anspruch 1 als Herbizide.

3. Herbizides Mittel, enthaltend flüssige und/oder feste Trägerstoffe und gewünschtenfalls mindestens ein Adjuvans sowie eine herbizid wirksame Menge mindestens eines substituierten 3-Phenylpyrazols der Formel I oder ein Salz von I, gemäß Anspruch 1.

4. Verfahren zur Herstellung von herbizid wirksamen Mitteln, dadurch gekennzeichnet, daß man eine herbizid wirksame Menge mindestens eines substituierten 3-Phenylpyrazols der Formel I oder ein Salz von I, gemäß Anspruch 1, mit flüssigen und/oder festen Trägerstoffen und gewünschtenfalls mindestens einem Adjuvans mischt.

5. Verfahren zur Bekämpfung von unerwünschtem Pflanzenwuchs, dadurch gekennzeichnet, daß man eine herbizid wirksame Menge mindestens eines substituierten 3-Phenylpyrazols der Formel I oder ein Salz von I, gemäß Anspruch 1, auf Pflanzen, deren Lebensraum oder auf Saatgut einwirken läßt.
